# EUROPÄISCHE PATENTSCHRIFT

(11) **EP 1 086 675 B1**
(45) Veröffentlichungstag und Bekanntmachung des Hinweises auf die Patenterteilung: **03.05.2006**
(21) Anmeldenummer: 00120181.3
(22) Anmeldetag: 22.09.2000
(51) Int. Cl.: A61F 13/08

(54) **Kompressionsmanschette zur Behandlung von Beinleiden**
Compression sleeve for the leg
Manchon de compression pour la contention de la jambe

(30) Priorität: 25.09.1999 DE 19946019; 27.09.1999 DE 29917030 U
(43) Veröffentlichungstag der Anmeldung: 28.03.2001
(73) Patentinhaber: Bauerfeind Orthopädie GmbH & Co. KG, 47880 Kempen (DE)
(72) Erfinder: Reinhardt, Holger, Dipl.-Ing., 47906 Kempen (DE); Bauerfeind, Hans B., 07937 Zeulenroda (DE)
(74) Vertreter: Bardehle, Heinz

(56) Entgegenhaltungen:
- CH-A- 465 762
- DE-A- 4 412 040
- DE-C- 463 086
- US-A- 2 672 139
- US-A- 3 306 288
- US-A- 4 862 523
- US-A- 4 977 011
- US-A- 5 904 710

## Beschreibung

Die Erfindung bezieht sich auf eine Kompressionsmanschette aus elastischem, textilen Grundmaterial zur Behandlung von Beinleiden.

Derartige Kompressionsmanschetten werden meist zusammen mit einem Fußteil verwendet, so daß es sich in diesem Falle um einen Kompressionsstrumpf handelt. Um mit derartigen Kompressionsstrümpfen einen besonders hohen Druck auf das zu behandelnde Bein zu erzeugen, hat man bereits gemäß dem DB GBM 29001000.8 mehrere Kompressionsstrümpfe übereinander angezogen, wodurch sich eine entsprechende Druckerhöhung ergibt. Bei der Behandlung von Beinleiden durch Kompressionsstrümpfe sind einerseits die Maße des zu behandelnden Beins des betreffenden Patienten und der jeweils erforderliche spezielle Kompressionsdruck zu berücksichtigen. Dabei handelt es sich normalerweise um Kompressionsmanschetten mit daran angebrachtem Fußteil, welche Kombination dann den vollständigen Kompressionstrumpf bildet, in dem allerdings die gewollte therapeutische Wirkung von seiner Kompressionsmanschette ausgeht. Um diesen Erfordernissen zu genügen, werden Kompressionsstrümpfe in verschiedenen Größen und unterschiedlichen Materialien hergestellt, wobei aufgrund der unterschiedlichen Materialien jeweils ein besonderer gewünschter Kompressionsdruck erzielbar ist. Hierfür ist eine erhebliche Anzahl von entsprechend unterschiedlich ausgebildeten Kompressionsstrümpfen erforderlich, was zu einem entsprechend hohen fabrikatorischen Aufwand führt, da normalerweise für die Berücksichtigung unterschiedlicher Anforderungen eine entsprechend große Anzahl von unterschiedlichen Kompressionsstrümpfen zur Verfügung stehen muß.

In der US 4,089,064 A ist eine Strumpfhose aus einem elastischen Grundmaterial offenbart, die in ihrem Kniebereich und Bereichen darunter und darüber einen Überzug aufweist, der durch ein nach Art eines Rhombenmusters gestaltetes unelastisches Textilmaterial gebildet wird. Bezüglich besonderer therapeutischer Effekte ist in der Druckschrift nichts offenbart, es wird nur darauf hingewiesen, dass damit eine zusätzliche Stütze für das Knie geschaffen werden soll. Auf jeden Fall erfordert der aus unelastischem Material bestehende Überzug die Bereitstellung einer größeren Anzahl von unterschiedlichen Größen der Strumpfhose, um diese für jeweils dickere oder dünnere Beine tragbar zu machen.

Weiterhin ist in der US 4,862,523 A ist eine aus einem elastischen Grundmaterial bestehende Strumpfhose offenbart, die zur Vermeidung eines Herabrutschens der Hose am Körper des Trägers mit im Beinbereich zwei ebenfalls elastischen Bändern versehen ist, die nach Art eines Gewindes sich kreuzen und dabei zwischen sich großflächige Rhomben einschließen, die sich jeweils über eine ganze Beinseite flächig erstrecken. Weiterhin sollen die beiden elastischen Bänder das Durchhängen des weichen Gewebes der unteren Gliedmaßen verzögem und eine auf das weiche Gewebe des Gesäßes und der Beine nach oben gerichtete Spannung aufrecht erhalten.

Der Erfindung liegt die Aufgabe zugrunde, eine Kompressionsmanschette auszubilden, die im Wesentlichen in einer Größe sowohl eine Anpassungsmöglichkeit an unterschiedliche Beingrößen und außerdem die Einstellung eines jeweils gewünschten individuellen Kompressionsdrucks ermöglicht. Dieses Problem wird im Zusammenhang mit der eingangs genannten Kompressionsmanschette dadurch gelöst, dass in das Grundmaterial ein Rhombengitter, bestehend aus in Umfangrichtung jeweils mehreren Rhomben pro Beinseite, mit in Manschettenlängsrichtung liegenden Diagonalen der Rhomben und mit das Rhombengitter bildenden, durchgehend schräg zur Manschettenlängsrichtung schraubenlinienartig verlaufenden, elastischen, sich kreuzenden Streifen integriert ist, deren Elastizität derart geringer ist als das von den Streifen eingeschlossene Grundmaterial, dass die durch Längsstrecken der Manschette jeweils gegebene Länge der Manschette deren Kompressionsdruck bestimmt.

Das in das elastische, textile Grundmaterial integrierte Rhombengitter wirkt sich über die gesamte Länge der Kompressionsmanschette in der Weise aus, dass eine auf eine kurze Länge eingestellte Kompressionsmanschette zu einem relativ großen Durchmesser aufgeweitet ist, wobei deren Kompressionsdruck durch diesen Durchmesser bestimmt ist. Ist dagegen die Kompressionsmanschette auf eine größere Länge gestreckt, so weist sie einen entsprechend geringeren Durchmesser auf, so dass die Kompressionsmanschette ein relativ dünnes Bein versorgen kann. Ist dann ein besonders hoher Kompressionsdruck erforderlich, so lässt sich dieser durch ein Auseinanderziehen der Manschette erzielen. Dabei spielt die Funktion des Rhombengitters die entscheidende Rolle, da das Rhombengitter bei Verkürzung seiner in Manschettenlängsrichtung liegenden Diagonalen (Längsdiagonalen) der Rhomben sich entsprechend ausweitet, und zwar wegen damit einhergehender Verlängerung der Querdiagonalen, die sich in Umfangsrichtung der Kompressionsmanschette erstrecken. Wird im Gegensatz dazu die Kompressionsmanschette in ihrer Längsrichtung gestreckt, so verkürzen sich die vorstehend erwähnten Querdiagonalen der einzelnen Rhomben, womit sich die Kompressionsmanschette zusammenzieht und einen entsprechend erhöhten Druck auf das zu behandelnde Bein ausübt. Das erwähnte Rhombengitter ermöglicht es also, sowohl die Dimensionen des zu behandelnden Beins durch entsprechende Anpassung des Durchmessers der Kompressionsmanschette zu berücksichtigen, als auch wahlweise jeweils den Kompressionsdruck der Kompressionsmanschette einzustellen. Damit erstreckt sich die erfindungsgemäße Kompressionsmanschette über einen weiten Anwendungsbereich, der sowohl unterschiedlich dünne bzw. starke Beine als auch eine erhebliche Variation des jeweils erforderlichen Kompressionsdrucks bietet. Für die jeweils von Patient zu Patient ganz unterschiedlich gegebenen Verhältnisse kann also in einem weiten Umfang eine einzige Größe der erfindungsgemäßen Kompressionsmanschette verwendet werden, was deren Herstellung und deren Herstellungskosten erheblich vereinfacht bzw. verringert.

Die Verwendung eines scherenartigen Geflechts von Bändern für einen orthopädischen Strumpf für die Oberschenkelextension ist an sich bekannt und zwar aus der DE OS 44 12 040. Beim Gegenstand dieser Druckschrift handelt es sich darum, eine als Strumpf bezeichnete Manschette über den Oberschenkel zu ziehen, wobei die Manschette mit einer entsprechenden Verlängerung über das Ende des Beines (ggf. eines Beinstumpfes) hinausragt und hier einer Gewichtsbelastung ausgesetzt wird, woraufhin sich die Manschette im Bereich des Oberschenkels zusammenzieht und so fest an den Oberschenkel anlegt, daß die Manschette vom Oberschenkel nicht abgleiten kann. Dies soll die Einleitung von Zugkräften auf den Oberschenkel erleichtern, womit dann über die so in den Oberschenkel eingeleiteten Zugkräfte auf den Oberschenkel und damit auf das Hüftgelenk einwirken können. An irgendeinen besonders einstellbaren Kompressionsdruck ist dabei also nicht gedacht.

Das die vorstehend geschilderte Anpassungsfähigkeit der Kompressionsmanschette bedingende Rhombengitter kann man in das Grundmaterial einstricken. Normalerweise werden Kompressionsstrümpfe aus einem gestrickten textilen Grundmaterial hergestellt, so daß in Anwendung auf derartige Kompressionsstrümpfe deren Manschettenteil durch eine für das Stricken bzw. Wirken bekannte Musterung des Grundmaterials geschaffen wird. Eine bevorzugte Musterung läuft darauf hinaus, im Bereich der sich kreuzenden Streifen eine Maschenbildung zu verhindern, so daß sich diesen Stellen ein verkürzter Fadenverlauf ergibt, bei dem die durch eine Maschenbildung gegebene Elastizität fehlt, was nichts anderes bedeutet, als daß in dem Streifen an den betreffenden Stellen dessen Elastizität verringert ist. Die sich kreuzenden Streifen geringerer Elastizität bilden damit das Rhombengitter, bei dem an den Kreuzungsstellen der Streifen durch den bei der Einstellung verwendeten Strickvorgang sich der Effekt einstellt, daß das Rhombengitter im Bereich seiner Streifen je nach Längsstrecken der Manschette sich die Querdiagonalen der einzelnen Rhomben mehr oder minder zusammenziehen, was dann die Anpassung an die jeweilige Beingröße und die Einstellung des jeweils erforderlichen Kompressionsdrucks ermöglicht.

Eine weitere Möglichkeit der Integration des Rhombengitters besteht darin, daß dieses auf das Grundmaterial aufgeklebt oder aufgeschweißt ist. Dabei kann das Rhombengitter aus einem elastischen Folienmaterial bestehen, aus dem das Rhombengitter ausgestanzt bzw. ausgeschnitten ist, so daß ein aus sich kreuzenden Streifen bestehendes Rhombengitter vorliegt, das dann auf die Kompressionsmanschette aufzulegen ist, um dann ggf. durch Erhitzung aufgeklebt bzw. aufgeschweißt zu werden. Das hierfür verwendete Folienmaterial ist elastisch, dessen Elastizität ist allerdings geringer als diejenige des Grundmaterials.

Es ist zwar dennoch möglich, das Grundmaterial selbst zur Bildung der Streifen zu beeinflussen, und zwar derart, daß sich eine örtliche Verschweißung bzw. Verklebung des Grundmaterials im Bereich der Streifen ergibt, was dadurch möglich ist, daß man das Grundmaterial durch Strahlung unter Abdeckung durch eine entsprechende Maske beeinflußt, von der nur die Bereiche der Streifen frei gelassen werden, so daß sich in diesen Bereichen dann eine Erwärmung ergibt, die zur Verschweißung bzw. zur Verklebung des Grundmaterials im Bereich der Streifen führt.

Eine weitere Möglichkeit der Ausbildung des Rhombengitters besteht darin, einen verflüssigten Kunststoff auf das Grundmaterial aufzubringen, und zwar den sich kreuzenden Streifen des Rhombengitters folgend, woraufhin der verflüssigte Kunststoff ausgehärtet wird, womit sich im Bereich des ausgehärteten Kunststoffs dann das Rhombengitter mit einer entsprechend geringeren Elastizität ergibt.

Es sei darauf hingewiesen, daß die vorstehend beschriebene Kompressionsmanschette natürlich auch mit einem Fußteil versehen werden kann, womit in seiner Gesamtheit dann ein Kompressionsstrumpf entsteht.

In den Figuren ist ein Ausführungsbeispiel der Erfindung dargestellt. Es zeigen
- Fig. 1: einen Kompressionsstrumpf mit einer Kompressionsmanschette, in der das Rhombengitter ausgebildet ist, und zwar im ungespannten Zustand ohne Längsstreckung,
- Fig. 2: den gleichen Kompressionsstrumpf mit Längsstreckung,
- Fig. 3: die an einem Unterschenkel angebrachte Kompressionsmanschette mit einem Fußteil, also insgesamt einen Kompressionsstrumpf in der Streckung gemäß Fig. 2.

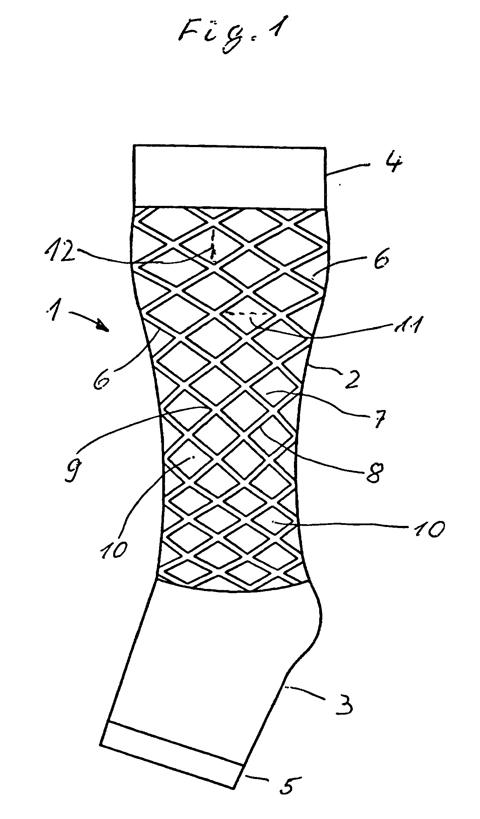

In der Fig. 1 ist der Kompressionsstrumpf 1, bestehend aus der Kompressionsmanschette 2 und dem Fußteil 3 dargestellt, wobei die Kompressionsmanschette 2 nach oben hin durch den Abschlußrand 4 begrenzt ist und das Fußteil 3 in dem Fußabschluß 5 endet. Kompressionsmanschette 2 und Fußteil 3 sind fest miteinander verbunden.

Die Kompressionsmanschette 2 besteht aus einem elastischen, textilen Grundmaterial, insbesondere einem Kompressionsgestrick, in die das Rhombengitter 6 gemäß diesem Ausführungsbeispiel eingestrickt ist. Das Rhombengitter 6 wird dabei durch die Streifen 7 und 8 gebildet, die sich jeweils schraubenlinienartig um die Kompressionsmanschette 2 erstrecken, und zwar jeweils entsprechend einem Rechts- und Linksgewinde. Hierdurch ergeben sich aufgrund der Kreuzungsstellen 9 der Streifen 7 und 8 einzelne Rhomben 10, die dann jeweils von vier Streifen 7 bzw. 8 eingeschlossen sind. Innerhalb dieser Rhomben 10 bildet das von den Streifen 7 und 8 eingeschlossene Grundmaterial den betreffenden Teil der Kompressionsmanschette. Die Streifen 7 und 8 sind so gestaltet, daß ihre Elastizität geringer ist als das von den Streifen 7 und 8 eingeschlossene Grundmaterial im Bereich jedes Rhombus. Es ergibt sich damit das aus der Fig. 1 ersichtliche Rhombengitter 6, das in der oben beschriebenen Weise auf die Kompressionsmanschette derart einwirkt, daß im Falle einer Längsstreckung der Kompressionsmanschette 2 sich die Querdiagonalen 11 zusammenziehen, während die Längsdiagonalen 12 aufgrund der Längsstreckung verlängert werden.

Es sei noch darauf hingewiesen, daß sich die in der Fig. 1 dargestellte Ansicht des Rhombengitters 6 natürlich umlaufend über die gesamte Kompressionsmanschette 2 erstreckt, so daß also ein von dem Rhombengitter 6 umfaßter vollständiger Schlauch in Form der Kompressionsmanschette 2 gegeben ist. Dabei können die Streifen auch fadenförmig ausgebildet sein.

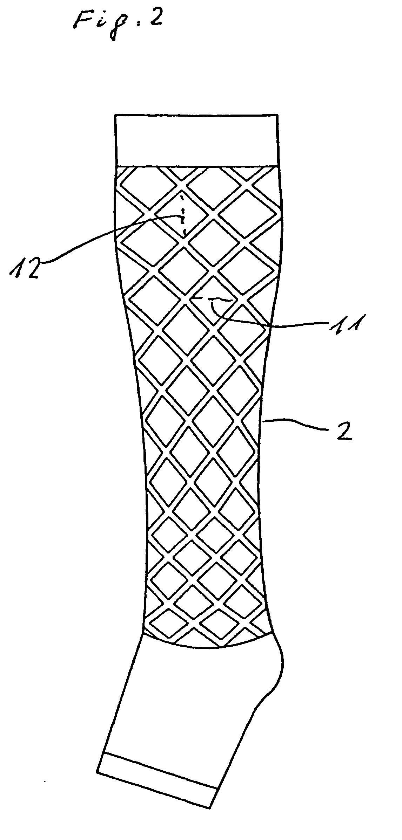

In der Fig. 2 ist die Kompressionsmanschette 2 gemäß Fig. 1 in einer längsgestreckten, längsgedehnten Lage wiedergegeben, wobei sich die Querdiagonalen 11 entsprechend verkürzt haben und die Längsdiagonalen 12 verlängert sind. Aufgrund dieser Längsstreckung der Kompressionsmanschette 2 ist ihre Länge insbesondere in Anpassung an die Länge eines Beines eines entsprechend großen Patienten verlängert.

Diese Art der Verkürzung bzw. Verlängerung der Kompressionsmanschette durch Längsstrecken bzw. Längsdehnen mit der dabei einhergehenden Verengung bzw. Erweiterung des Durchmessers der Kompressionsmanschette läßt sich natürlich über die in den Fig. 1 und 2 hinausgehenden Dimensionen erweitern, um auf diese Weise die Kompressionsmanschette an unterschiedlich starke Beine und unterschiedlich erforderliche Kompressionsdrücke anzupassen.

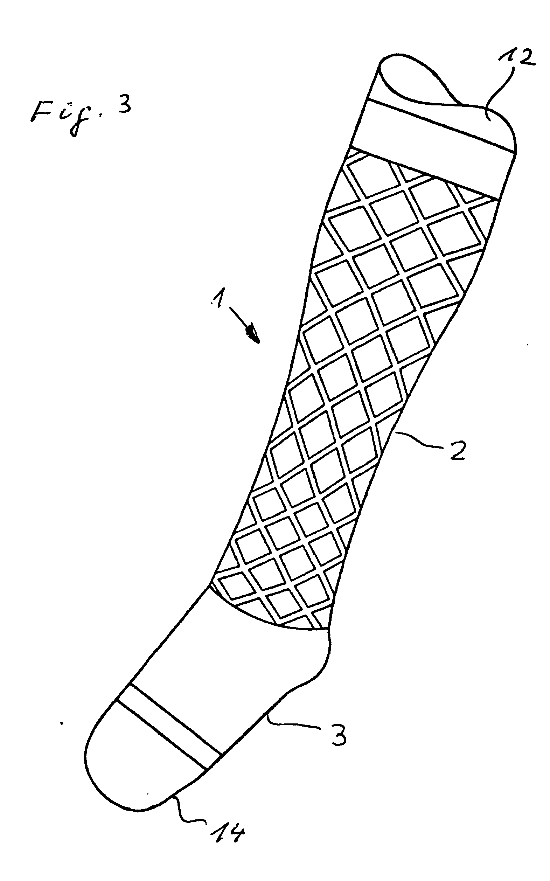

In der Fig. 3 ist die Kompressionsmanschette 2, wie sie in der Fig. 2 dargestellt ist, als ein an einem Unterschenkel 13 angebrachter Kompressionsstrumpf 1 dargestellt. Aus dem Fußteil 3 ragt der vereinfacht dargestellte Vorderfuß 14 hervor. Ein mit der erfindungsgemäßen Kompressionsmanschette 2 ausgestatteter Kompressionsstrumpf 1 kann in dieser Weise, angepaßt an die jeweiligen individuellen Verhältnisse, ohne weiteres auf jeden Unterschenkel aufgezogen werden, wobei das Rhombengitter aufgrund seiner Streckeigenschaften in einem weiten Umfang eine Anpassung des Kompressionsstrumpfes an ganz unterschiedliche Unterschenkelgrößen ermöglicht.

## Patentansprüche

1. Kompressionsmanschette (2) aus elastischem, textilen Grundmaterial zur Behandlung von Beinleiden, **dadurch gekennzeichnet, dass** in das Grundmaterial ein Rhombengitter (6), bestehend aus in Umfangrichtung jeweils mehreren Rhomben pro Beinseite, mit in Manschettenlängsrichtung liegenden Diagonalen (12) der Rhomben (10) und mit das Rhombengitter (6) bildenden, durchgehend schräg zur Manschettenlängsrichtung schraubenlinienartig verlaufenden, elastischen, sich kreuzenden Streifen (7, 8) integriert ist, deren Elastizität derart geringer ist als das von den Streifen (7, 8) eingeschlossene Grundmaterial, dass die durch Längsstrecken der Manschette (2) jeweils gegebene Länge der Manschette (2) deren Kompressionsdruck bestimmt.

2. Kompressionsmanschette nach Anspruch 1, **dadurch gekennzeichnet, dass** das Rhombengitter (6) in das Grundmaterial eingestrickt ist.

3. Kompressionsmanschette nach Anspruch 1, **dadurch gekennzeichnet, dass** das Rhombengitter (6) aufgeklebt ist.

4. Kompressionsmanschette nach Anspruch 1, **dadurch gekennzeichnet, dass** das Rhombengitter (6) aufgeschweißt ist.

5. Kompressionsmanschette nach Anspruch 3 oder 4, **dadurch gekennzeichnet, dass** das Rhombengitter (6) aus einem elastischen Folienmaterial besteht.

6. Kompressionsmanschette nach Anspruch 1, **dadurch gekennzeichnet, daß** das Rhombengitter (6) durch örtliche Verschweißung bzw. Verklebung des Grundmaterials im Bereich der Streifen ausgebildet ist.

7. Kompressionsmanschette nach Anspruch 1, **dadurch gekennzeichnet, daß** das Rhombengitter (6) als verflüssigter Kunststoff auf das Grundmaterial aufgebracht ist, der nach dem Aufbringen ausgehärtet ist.

8. Kompressionsmanschette nach einem der vorhergehenden Ansprüche, **dadurch gekennzeichnet, daß** sie in einem Fußteil (3) endet.

## Revendications

1. Manchon compressif (2) en matière de base textile élastique pour le traitement de maux de jambes, **caractérisé en ce que** dans la matière de base est intégrée une résille losangée (6) constituée de plusieurs losanges disposés dans la direction circonférentielle sur chaque côté de jambe, les diagonales (12) des losanges (10) étant orientées dans la direction longitudinale du manchon, et avec des bandes élastiques croisées (7, 8) qui forment la résille losangée (6), qui s'étendent en continu de manière hélicoïdale en biais par rapport à la direction longitudinale du manchon et dont l'élasticité est inférieure à celle de la matière de base enfermée par les bandes (7, 8), et **en ce que** la longueur du manchon (2) définie par allongement longitudinal de celui-ci détermine sa force de compression.

2. Manchon compressif selon la revendication 1, **caractérisé en ce que** la résille losangée (6) est intégrée par tricotage dans la matière de base.

3. Manchon compressif selon la revendication 1, **caractérisé en ce que** la résille losangée (6) est rapportée par collage.

4. Manchon compressif selon la revendication 1, **caractérisé en ce que** la résille losangée (6) est rapportée par soudage.

5. Manchon compressif selon la revendication 3 ou 4, **caractérisé en ce que** la résille losangée (6) est constituée d'une matière élastique en feuille.

6. Manchon compressif selon la revendication 1, **caractérisé en ce que** la résille losangée (6) est réalisée par soudage, respectivement collage local de la matière de base dans la zone des bandes.

7. Manchon compressif selon la revendication 1, **caractérisé en ce que** la résille losangée (6) est appliquée sur la matière de base sous forme de matière synthétique liquéfiée, laquelle est ensuite durcie.

8. Manchon compressif selon une des revendications précédentes, **caractérisé en ce qu'**il se termine par une partie de pied (3).

## Claims

1. Compression sleeve (2) made of elastic base material for the treatment of leg complaints, **characterised in that** a rhombic lattice (6) consisting of several rhombuses on each side of the leg in the circumferential direction is integrated into the base material, with diagonals (12) of the rhombuses (10) lying in the longitudinal direction of the sleeve and with intersecting elastic strips (7, 8) extending helically and continuously obliquely relative to the longitudinal direction of the sleeve and forming the rhombic lattice (6), the elasticity of these strips being lower than that of the base material enclosed by the strips (7, 8), in such a manner that the length of the sleeve (2) resulting from longitudinal stretching of the sleeve (2) determines the compression thereof.

2. Compression sleeve according to claim 1, **characterised in that** the rhombic lattice (6) is knitted into the base material.

3. Compression sleeve according to claim 1, **characterised in that** the rhombic lattice (6) is stuck on.

4. Compression sleeve according to claim 1, **characterised in that** the rhombic lattice (6) is welded on.

5. Compression sleeve according to claim 3 or claim 4, **characterised in that** the rhombic lattice (6) consists of an elastic film material.

6. Compression sleeve according to claim 1, **characterised in that** the rhombic lattice (6) is formed by local welding or bonding of the base material in the region of the strips.

7. Compression sleeve according to claim 1, **characterised in that** the rhombic lattice (6) is applied to the base material in the form of liquefied plastic and is hardened after application.

8. Compression sleeve according to one of the preceding claims, **characterised in that** it ends in a foot part (3).
